# FASCICULE DE BREVET EUROPEEN

(11) **EP 1 082 068 B1**
(45) Date de publication et mention de la délivrance du brevet: **04.06.2008**
(21) Numéro de dépôt: 99923672.2
(22) Date de dépôt: 04.06.1999
(51) Int. Cl.: A61F 2/02

(54) **DISPOSITIF INTRALUMINAL IMPLANTABLE**
IMPLANTIERBARE INTRALUMINALE VORRICHTUNG
IMPLANTABLE INTRALUMINAL DEVICE

(30) Priorité: 04.06.1998 FR 9807036
(43) Date de publication de la demande: 14.03.2001
(73) Titulaire: SOCIETE DE CONSEILS DE RECHERCHES ET D'APPLICATIONS SCIENTIFIQUES (S.C.R.A.S.), 75016 Paris (FR)
(72) Inventeur: CHERIF-CHEIKH, Roland, F-92130 Issy-les-Moulineaux (FR)
(74) Mandataire: Bernasconi, Jean Raymond
(86) Numéro de dépôt international: PCT/FR1999/001324
(87) Numéro de publication internationale: WO 1999/062426

(56) Documents cités:
- WO-A-90/04982
- WO-A-91/17744
- WO-A-98/58599
- DE-U- 29 615 067
- US-A- 5 695 518

## Description

La resténose vasculaire est le processus pathologique par lequel les vaisseaux sont obstrués et qui correspond, notamment, aux maladies coronaires. Il est lié à l'élargissement ou l'épaississement de la paroi interne des artères qui provoque le rétrécissement de la lumière vasculaire et la diminution du débit ou flux sanguin. Le même processus est impliqué dans les athéroscléroses artérielles, notamment cérébrales ou relatives aux membres, notamment aux membres inférieurs.

Ces maladies peuvent de nos jours faire l'objet d'un traitement par cardiologie interventionnelle au lieu de la chirurgie cardiaque plus ancienne. La cardiologie interventionnelle consiste à introduire, sous anesthésie locale, dans l'artère fémorale un cathéter d'angioplastie. Sous contrôle radiologique, on place ce cathéter au niveau de la sténose.

Plusieurs types de cathéters sont utilisés pour réaliser une angioplastie, notamment les cathéters à ballonnets ou PTCA (Percutaneous Transluminal Coronary Angioplasty); les cathéters DCA ou RCA (Directional ou Rotational Coronary Atherectomy) ou les cathéters LA (Laser Angioplasty).

Ces techniques sont peu invasives, ce qui permet de réduire la durée de séjour hospitalier.

L'inconvénient majeur est le taux de resténose à trois mois qui atteint 30 à 50%. Pour tenter d'éviter un pontage, qui serait alors souvent inévitable, on utilise aujourd'hui des implants écarteurs ou stents.

De nombreux systèmes écarteurs ou stents sont déjà utilisés. Il s'agit en général d'une structure (maillage ou ressort) réalisée en métal et susceptible de venir s'expanser en forme de cylindre creux à l'intérieur d'un vaisseau.

US-A-5,695,518, DE 29615067 et W098158599 décrivent des dispositifs implantables dans les voies vasculaires, comportant un élément intraluminal allongé susceptible d'occuper, dans un tronçon de la lumière vasculaire, un volume de cette lumière, ledit élément étant associé à un moyen de fixation dans la lumière vasculaire.
Le document WO 98/58599 est un état de la technique selon l'article 54(3) CBE.

En principe, les stents, placés après la procédure d'angioplastie au niveau de la paroi à traiter, sont censés diminuer le taux de resténose à trois mois. La principale raison est que les stents augmentent très nettement le diamètre de la lumière vasculaire. La même resténose des vaisseaux ne suffit plus à obstruer le vaisseau pour une même période après l'intervention et même au-delà, car le diamètre initial de la lumière est plus important.

La resténose résulte de divers facteurs importants parmi lesquels on peut citer la réaction inflammatoire, l'élasticité des parois vasculaires, la thrombose murale ou la prolifération des cellules du muscle lisse.

Certains résultats cliniques récents obtenus avec des stents permettent de penser que le taux réel de resténose n'est pas diminué. Si pour une même période de temps après l'intervention, on évite l'obstruction, il est possible que le stent ne fasse que retarder l'échéance. Avec le recul du temps dans l'utilisation des stents, on note, en effet, un taux croissant de resténose tardive devant conduire à la réintervention.

Face à cette menace, on peut associer aux stents l'utilisation d'un principe actif, par exemple, par recouvrement du stent, dans le but d'éviter la resténose sous l'action combinée, mécanique du stent et pharmacologique du produit.

Cette approche se heurte à différentes difficultés parmi lesquelles la très faible surface du stent, qui ne permet pas l'association d'une quantité importante de principe actif, ou la localisation du produit entre le stent et la paroi du vaisseau, qui, soit ne correspond pas à la zone optimale de relargage, soit entraîne son élimination rapide dans le flux sanguin.

Une autre difficulté majeure de cette solution est qu'il n'existe pas de produit ayant vraiment fait la preuve d'une efficacité antiresténose, surtout au niveau local intravasculaire.

Les résultats décevants obtenus à terme avec les stents pourraient s'expliquer par le fait que ce dispositif mécanique n'agit pas sur la cause de la resténose mais cherche simplement à éviter sa conséquence.

Les différentes techniques d'angioplastie PTCA, DCA, LA ou encore ultrasons sont toutes des techniques traumatiques de revascularisation.

Il s'agit en fait d'écarter les tissus vasculaires, d'éliminer les plaques d'athérome qui seront écrasées, détruites ou retirées pour rétablir le trajet sanguin. La zone traitée est donc une zone lésionnelle susceptible d'entraîner des phénomènes d'adhésion post-chirurgicale, des cicatrices et des formations tissulaires qui pourraient expliquer plus tard la resténose.

Il existe de plus un risque de thrombose dans cette zone après la lyse et la dilatation tissulaire.

L'implantation d'un stent directement au contact de cette surface lésionnelle pourrait entraîner des phénomènes d'inflammation chronique et créer des problèmes d'agrégation. Il s'agit, de plus, d'un corps étranger qui est directement mis en contact avec des tissus sur lesquels il exerce une contrainte mécanique permanente. Ces tissus intravasculaires ne sont pas très tolérants aux corps étrangers.

De nombreux facteurs peuvent expliquer les problèmes d'artériosclérose après la pose d'un stent.

Le risque thrombogénique et inflammatoire est augmenté par l'utilisation de toute structure creuse, de surface non lisse, qu'elle soit en contact avec le courant sanguin dans le flux central ou à sa périphérie.

En dehors du traitement de gros vaisseaux coronaires, il existe avec l'amélioration des stents et des techniques d'accès vasculaire, de plus en plus d'applications radiologiques pour les maladies périvasculaires.

Tous les phénomènes tissulaires ou cellulaires sont alors amplifiés par la diminution de la lumière du vaisseau qui augmente le risque de resténose.

Il est, d'autre part, plus difficile de réaliser et d'utiliser efficacement un stent sur des trajets vasculaires longs et, ou étroits, avec plus de risques d'échec. Si le trajet vasculaire à traiter n'est pas rectiligne ou s'il existe une ramification, la pose d'un stent est également plus délicate, voire impossible.

La resténose post-angioplastie peut conduire à une obturation nouvelle qui permet d'envisager à nouveau tous les traitements de cardiologie interventionnelle. Au contraire, la réaction de prolifération post-opératoire des tissus, si elle a lieu autour d'un stent, va emprisonner celui-ci et rendre très compliqué, voire impossible selon la taille du vaisseau, le recours à tout autre traitement que la chirurgie de pontage.

La présente invention se propose de fournir un dispositif qui évite ou limite les inconvénients de l'art antérieur et qui permette de diminuer les risques de sténose, ou de resténose, notamment après angioplastie.

Un autre objectif est de fournir un tel dispositif qui soit d'une grande simplicité de fabrication et d'un coût réduit.

Un autre objectif de l'invention est de fournir un tel dispositif qui soit facile à mettre en place et à fixer à l'emplacement voulu dans le trajet vasculaire à préserver.

Un autre objectif de l'invention est de réaliser un tel dispositif qui puisse être beaucoup plus facilement retiré qu'un stent.

Un autre objectif de l'invention est de fournir un tel dispositif qui puisse délivrer une quantité importante d'un principe actif présentant une efficacité contre la sténose ou la resténose.

Un autre objectif encore est de fournir un dispositif qui soit résorbable et puisse disparaître de lui-même, après un certain temps.

Un autre objectif, encore, de l'invention est de fournir un tel dispositif qui limite ou supprime une action mécanique sur la paroi du vaisseau.

Un autre objectif de l'invention est de fournir un tel dispositif qui puisse être facilement fabriqué selon des dimensions différentes et, avantageusement en permettant d'utiliser un même dispositif de dimensions déterminées dans une plage relativement large de diamètre luminal.

La présente invention a pour objet un dispositif implantable dans les voies vasculaires, tel que définit dans les revendications.

Ce dispositif se présente, de préférence, comme une tige, de préférence, pleine et lisse ou régulière dans sa surface longitudinale, de diamètre et de section, de préférence, constants, dont une des extrémités est libre; l'autre étant, soit retenue par contact avec la paroi du vaisseau, soit implantée dans cette paroi, par ledit moyen de fixation; ceci directement ou par l'intermédiaire d'une zone d'ancrage associée.

L'objectif de ce dispositif est de modifier l'influence de la circulation vasculaire exercée en local sur la paroi traitée sans diminuer le flux sanguin et sans action mécanique directe sur cette paroi du vaisseau. Ce dispositif permet plus particulièrement d'augmenter la vitesse du flux sanguin au niveau de la paroi vasculaire. Cette modification sans diminution du débit est rendue possible par l'élasticité du vaisseau à laquelle participent l'endothélium et l'intima.

Bien que l'on n'ait pas actuellement une explication certaine de l'efficacité du dispositif selon l'invention, on pense que la réaction qui suit l'installation d'un dispositif d'écartement tel qu'un stent, conduira à la resténose tardive en raison de l'augmentation de l'épaisseur de l'intima du vaisseau.

Cette intima hyperplasique (IH) est contrôlée par l'endothélium. Il semble que cette prolifération diminue si le flot sanguin est important et augmente si ce flot est faible. Dans le cas de l'utilisation d'un stent, la distension du tissu qu'il engendre pourrait inhiber cette médiation de la prolifération. En effet, les tissus vasculaires au niveau local sont alors soumis à une extension mécanique, qui malgré l'augmentation du flux obtenu par le stent, ne reflète pas la pression sanguine normale à ce site sur la paroi vasculaire.

Tout dispositif qui viendrait s'appuyer dans le vaisseau sur une grande surface comme un stent sur la zone artériosclérosique, constitue ainsi une surface de distension mécanique qui pourrait donc à terme empêcher la régulation de la prolifération de l'intima et présenter un risque de resténose.

La resténose post-angioplastique est donc un remodelage ou une hyperplasie de l'intima. Pour l'éviter, il convient donc d'inhiber cette prolifération et migration des cellules du muscle lisse.

Ce désordre vasculo-prolifératif est un processus relativement lent qui va mettre trois mois au moins pour obstruer un vaisseau (et beaucoup plus s'il y a un stent).

On dispose donc, dans tous les cas, d'une période de temps pendant laquelle, à la suite de l'angioplastie, le flot sanguin est rétabli.

Le dispositif selon l'invention va permettre sur cette période essentielle, et éventuellement au-delà de modifier la circulation sanguine sur la zone ayant subi la procédure de revascularisation interventionnelle, sans aucun écartement ni contact direct avec la paroi lésée. Ainsi, l'augmentation du flot sanguin pourra pleinement jouer son rôle freinateur sur la prolifération de l'intima et éviter la resténose. On propose ainsi un stimulus à la régulation biologique en évitant le dispositif mécanique de contact qui pourrait perturber cette régulation.

Le moyen proposé par le dispositif de l'invention est d'installer dans cette partie du courant sanguin un dispositif qui va occuper un volume et donc, forcer le flux nouvellement rétabli à exercer une action sur la paroi du vaisseau qui permettra au mécanisme biologique de réguler la prolifération cellulaire pour éviter la resténose.

Une des caractéristiques importantes du dispositif est donc d'être un "non stent" c'est-à-dire d'éviter toute action mécanique sensible sur la paroi du tronçon affecté du vaisseau, susceptible d'inhiber l'efficacité régulatrice de la pression sanguine et, de préférence, tout contact permanent.

Ainsi, de façon plus avantageuse, le dispositif pourra être retenu à la surface ou ancré dans la paroi du vaisseau en amont de la zone à traiter de telle sorte que seul l'élément ou tige dans le courant sanguin se trouve dans la zone traumatique sans aucun contact sensible ou même aucun contact avec la paroi.

Le dispositif pouvant être constitué d'une tige pleine, il est très facile d'établir et de contrôler le volume occupé par rapport au diamètre des vaisseaux et ainsi, de réguler les paramètres de circulation sanguine.

Pour un vaisseau de diamètre de lumière donnée, plus le dispositif sera de grand diamètre, plus la vitesse sanguine augmentera dans une marge de proportion donnée liée à l'élasticité et aux capacités de réaction dans des couches cellulaires qui composent le vaisseau. Au-delà, le dispositif pourrait en effet diminuer le flux sanguin.

Pour diminuer ce risque, cette tige pleine sera préférentiellement réalisée avec une surface lisse dans le sens longitudinal, qui favorisera l'écoulement du sang. Par rapport au maillage ou à des dispositifs creux, ce dispositif plein et lisse permet aussi de réduire les risques de thrombose ou d'agrégation.

Cette forme pleine et lisse permet donc d'envisager un volume supérieur à une forme creuse ou à une forme de surface irrégulière sans risque de diminuer le flux.

Le dispositif, occupera, de préférence, un volume tel que sa surface de section puisse présenter entre 5 et 50% de la surface de la lumière du vaisseau. Ce pourcentage pourra être adapté en fonction de l'importance de la sténose, de son traitement et de sa longueur. Ce pourcentage pourra varier en fonction de l'utilisation (coronaire ou vaisseau périphérique).

De préférence, la section transversale du dispositif est comprise entre 10 et 20% de la section du vaisseau où il est implanté. Cette section peut être cylindrique, ovale, carrée ou avoir tout autre forme, par exemple, une forme en étoile aux extrémités angulaires et arrondies aux creux de façon à diminuer la surface de contact possible avec la paroi vasculaire tout en favorisant l'écoulement périphérique du sang.

De préférence, le dispositif selon l'invention possède un diamètre compris entre 0,5 et 2 mm.

Sa longueur est de préférence comprise entre 0,5 et 15 cm de longueur.

La structure allongée du dispositif peut être rigide, semi-rigide ou souple, notamment lorsqu'elle possède une grande longueur.

Le système d'ancrage dans la paroi du vaisseau, par contact ou implanté, permet d'éviter l'appui ou le support sur le vaisseau dans la zone à traiter. Il peut s'agir d'un ancrage en plusieurs points ou circulaire mais préférentiellement deux points d'ancrage pour un ancrage par contact, ou un seul point d'ancrage pour l'implantation, seront retenus de façon à perturber le moins possible le flux sanguin et les réactions de la paroi vasculaire. De toute façon, même dans le cas d'une retenue par contact, il ne s'agira que d'un appui faible et non d'un étirement mécanique comme pour les stents.

Cet ancrage peut être obtenu par extension, dans la lumière du vaisseau, d'une zone d'ancrage en amont de l'élément ou tige du dispositif qui va s'écarter pour toucher la paroi du vaisseau au moins en deux points, lorsqu'elle est libérée par un système d'administration tel que, par exemple, une aiguille creuse ou un cathéter, le dispositif étant contraint de garder une forme rectiligne dans sa zone d'ancrage, tant qu'il n'a pas été libéré.

Cet écartement sera plus ou moins important selon le diamètre de la lumière; ce qui rendra le dispositif utilisable quelque soit le diamètre du vaisseau.

De plus, plutôt que l'extension, c'est, en général, le blocage, en travers du flux, de la zone d'ancrage contrôlée par l'extrémité libre de la tige qui va assurer le positionnement.

La pose et la libération du dispositif dans sa zone d'insertion pourra être assurée par des systèmes équivalents à ceux utilisés pour positionner les stents, c'est-à-dire des systèmes de pose avec des cathéters interventionnels et, ou des câbles guides actifs.

La translation de l'un de ces éléments, par rapport à l'autre, libérera le dispositif. Le dispositif pouvant être filiforme, le diamètre total du cathéter pourra donc être encore inférieur à ceux des cathéters utilisés pour les stents.

Un tel mécanisme de libération de l'ancrage peut également être utilisé pour permettre l'implantation du moyen d'ancrage dans la paroi du vaisseau.

Ce type d'ancrage peut aussi être obtenu par injection transluminale d'une aiguille contenant le dispositif avec éventuellement une zone d'ancrage. Ce système d'insertion s'apparente à celui décrit dans les demandes FR 96 14755 et PCT FR 97102182. L'aiguille, le dispositif, et la tige guide d'activation seront installés dans un cathéter interventionnel équivalent à ceux utilisés pour placer les stents. Le dispositif peut aussi être une partie du guide qui restera en place.

L'aiguille peut être piquée à travers la paroi du vaisseau, le guide ou mandrin permettant alors de libérer le dispositif ou son moyen d'ancrage, dans la zone périphérique du vaisseau. L'aiguille est alors retirée de la paroi puis le cathéter interventionnel libérera ainsi, dans le courant sanguin, le dispositif fixé.

Ledit dispositif sera alors préférentiellement suffisamment souple ou flexible pour être positionné par le courant sanguin.

Le mécanisme de libération du dispositif ou de son ancrage peut être commandé de l'extérieur par les mêmes systèmes de transmission qui libèrent un stent au bout du cathéter c'est-à-dire le retrait du cathéter ou l'avancée du guide ou mandrin.

Selon un autre mécanisme d'insertion, le dispositif est lui-même réalisé sous forme d'une aiguille pleine à une de ses extrémités correspondant à la zone d'ancrage qui peut être injectée dans la paroi par retrait d'un cathéter guide. Si le dispositif est souple ou flexible ce retrait entraîne ensuite la libération de l'élément allongé du dispositif jusqu'à l'autre extrémité. Si le dispositif est plus rigide, il peut être libéré par le cathéter-guide avant l'implantation de la zone d'ancrage.

L'extrémité pointue en forme d'aiguille qui constitue le moyen d'ancrage peut être non rectiligne par rapport à la tige dans le flux sanguin de façon à favoriser son implantation et le positionnement de la tige libre en aval dans le vaisseau. Une forme en harpon, en hameçon avec un ardillon peut assurer le maintien de la zone d'ancrage dans la paroi vasculaire.

Le dispositif peut également être simplement rectiligne et planté dans la paroi du vaisseau où il sera retenu par la seule pression des tissus et par la cicatrisation. On peut envisager que la partie d'ancrage ne soit pas lisse mais présente des aspérités qui favorisent son maintien dans les tissus. L'alignement dans le courant de l'élément souple va entraîner un angle aigu par rapport à son insertion, qui favorisera son maintien.

Le moyen d'ancrage peut s'expanser pour assurer son implantation dans la paroi à partir du retrait du cathéter guide.

S'agissant d'un dispositif non creux, le noyau ou mandrin (guide wire) n'est plus nécessaire, l'ensemble dispositif système d'implantation peut donc être plus fin que l'ensemble stent et système d'implantation. Le cathéter d'intervention sera donc de diamètre réduit, ce qui permet une intervention plus facile et l'accès à des sites plus étroits qu'avec les dispositifs de type stent.

Pour traiter une zone en arborisation, avec deux ou plusieurs voies et quelle que soit la forme, on pourra avoir recours à l'implantation de plusieurs dispositifs préférentiellement souples ou flexibles dans le tronc commun dont chacun aura son extrémité placée à l'intérieur d'une des voies à traiter. Le dispositif s'adapte ainsi à tous les arrangements vasculaires beaucoup plus facilement que les stents et sans nécessiter de formes spécifiques.

Le dispositif pouvant être installé juste après l'angioplastie, son action favorable sur la pression pourra intervenir dès le début et sur toute la période à risque de resténose.

Il est possible d'envisager différentes formes de dispositif que l'on souhaitera ou non, retirer ou éliminer après son action.

Dans certains cas, après une période de 3 à 6 mois, il pourra être souhaitable de voir disparaître le dispositif.

La conséquence de ce retrait ou de cette élimination pourrait être une variation de la pression locale, mais celle-ci sera sans effet sur une zone stabilisée et de plus compensée ou diminuée par l'élasticité du vaisseau.

Dans le cas d'un retrait, étant donné la forme, de préférence, lisse et pleine du dispositif et sa position sans écartement de la paroi, on évite tous les problèmes liés à la resténose autour d'un stent et, même en cas de resténose, le retrait reste simple et conduit instantanément à une recanalisation qui permet de nouveau tous les traitements d'angioplastie.

Ce retrait pourra être pratiqué à partir d'une voie endovasculaire en venant saisir ou fixer la zone d'ancrage (contact ou insertion, par exemple, avec une pince ou un crochet) puis extraction ou encore à partir d'une voie périvasculaire également en tirant sur la zone d'ancrage.

L'angle de positionnement du moyen d'ancrage, par contact ou insertion, peut permettre un retrait facile simplement en tirant sur la zone d'ancrage. Il est possible de prévoir, en amont de la zone d'ancrage, une zone de fixation pour retrait ultérieur.

Le dispositif peut être constitué, par exemple, d'un fil d'acier inoxydable de diamètre constant. II pourra être réalisé avec les matériaux classiquement utilisés pour les stents et notamment les métaux et leurs alliages.

Le dispositif pourra donc agir sur une longueur plus importante que les stents et être positionné dans le vaisseau quelque soit leur diamètre et leur forme. En fonction des matériaux utilisés, de son diamètre et de sa souplesse, il s'adaptera, en effet, au trajet vasculaire.

Etant donné le diamètre très faible et l'encombrement du dispositif, on peut, au niveau d'une ramification vasculaire, procéder à l'implantation de plusieurs dispositifs avec un seul ou deux cathéters interventionnels. Le cathéter unique pourra, par exemple, libérer deux tubes guides qui s'écarteront dans chacune des vois vasculaires permettant la libération de la tige libre des dispositifs suivi de la libération des deux moyens d'ancrage dans le tronc commun.

On pourra réaliser le dispositif en plastique puisqu'aucune contrainte mécanique ne s'exerce sur ce dispositif. Il pourra, par exemple, être fait avec les matériaux des fils de suture non biodégradables, en Nylon ou en polysulfone.

Le dispositif peut être réalisé avec tous les biomatériaux susceptibles d'améliorer sa tolérance locale, par exemple, en Téflon (PTFE), en silicone...

On peut également le réaliser avec les matériaux de fils de suture biodégradables, c'est-à-dire, par exemple, en PLGA (polylactide co-glycolide).

L'avantage d'utiliser le PLGA est d'obtenir un dispositif dont la durée de vie dans le courant sanguin est connue (par exemple 3 mois) et qui sera naturellement éliminé ensuite sans aucune intervention de retrait.

Un autre avantage possible à l'utilisation du PLGA ou d'un autre matériau biodégradable est la possibilité d'associer au dispositif un principe actif (PA) susceptible d'éviter ou de réduire les risques de resténose.

L'ensemble du volume du dispositif (y compris éventuellement le système d'ancrage) pourra être utilisé comme réservoir de formulation PLGA de façon à avoir à la fois une diffusion du PA endo et périvasculaire.

En dehors des biomatériaux biodégradables ou non, plastiques ou métalliques utilisables pour ce dispositif, il est possible d'avoir recours à un système de revêtement, hydrophile ou autre, pour améliorer la biocompatibilité ou rendre la surface de l'élément allongé antiadhésive. Par exemple, on pourra recouvrir le dispositif d'acide hyaluronique, de collagène, de PEG (polyéthylène glycol), de glycérol, d'hydrogel etc. On pourra également procéder à une siliconisation ou à un épilamage de la surface métallique.

On pourra utiliser également tous les procédés de modifications de surface imaginés pour les stents.

Il est également possible de réaliser un revêtement d'un dispositif métallique ou plastique avec un PLGA qui pourra contenir un PA; par exemple le Lanréotide, l'hydrocortisone, un glucocorticoïde, un produit cystostatique (anticancéreux) ou tous les PA envisagés avec les stents.

On obtiendra de la sorte, l'effet local et systémique du produit et on diminuera le diamètre du dispositif donc son effet sur la circulation locale après élimination du revêtement PLGA ou de tous autres excipients retard.

Etant donné la forme des dispositifs, ils pourront avantageusement être fabriqués par extrusion ou filage en utilisant exactement les mêmes techniques que celles permettant de réaliser les cathéters, tubes, mandrins, fils de suture, etc. La forme de la filière utilisée et l'étirage détermineront la section constante et la forme du dispositif (rond, carré ou en étoile).

Le coût de fabrication de ce dispositif sera donc extrêmement réduit; ce qui permet une plus large utilisation et la réalisation de modèles spécifiques à chaque cas.

Le dispositif selon l'invention peut être implanté par un procédé d'implantation qui met en oeuvre un cathéter interventionnel du même type que ceux utilisés pour la pose des stents, comme décrit ci-dessus. Ce cathéter peut, le cas échéant, être retiré par des moyens externes libérant la zone d'ancrage, puis l'ensemble du dispositif jusqu'à la zone d'ancrage.

La zone d'ancrage peut, pour la pose, être implantée par une aiguille disposée à l'intérieur du cathéter.

Le dispositif selon l'invention trouve son application dans un procédé thérapeutique consistant, à la suite d'une angioplastie, à installer dans le courant de la lumière vasculaire, un dispositif tel que décrit ci-dessus dont le volume va entraîner une variation de la circulation liquidienne locale sans que ce dispositif n'interfère, par contact, avec les parois vasculaires.

Ce procédé permet donc de créer un stimulus de régulation sur la prolifération vasculaire dans les tronçons de vaisseaux qui ont été soumis à l'angioplastie, ce qui permettra d'éviter la resténose vasculaire.

L'implantation peut être effectuée de façon que les moyens d'ancrage soient situés en amont de l'élément allongé du dispositif dans le sens du flux, ou au contraire, en aval, l'extrémité libre de l'élément allongé étant alors disposée en position amont.

De préférence, la fixation, par une zone d'ancrage, s'effectuera en dehors du tronçon proprement dit, qui a été soumis à l'angioplastie. Cependant, du fait que les moyens de fixation n'exercent pas d'action mécanique sensible sur la paroi vasculaire, si on la compare à l'effet d'un stent ou dispositif similaire, on peut également fixer le dispositif au niveau d'un tronçon qui a été soumis à une angioplastie.

Dans une forme perfectionnée du procédé on peut retirer, après l'utilisation, ce dispositif en libérant les moyens de fixation, par exemple à l'aide d'un dispositif de reprise dans un cathéter.

Cependant, dans un autre mode du procédé, on peut laisser le dispositif se résorber lorsqu'il est réalisé en un matériau convenable jusqu'à finir par disparaitre.

Le procédé peut comprendre, concomitamment, une délivrance d'un principe actif susceptible de s'opposer à la resténose et, de façon avantageuse, ce principe actif peut être porté et libéré par le dispositif lui-même.

D'autres avantages et caractéristiques de l'invention apparaîtront à la lecture de la description suivante, faite à titre d'exemple non limitatif, et se référant aux dessins annexés dans lesquels :
- la figure 1 représente une vue schématique en section longitudinale d'un dispositif en cours d'implantation par un cathéter ;
- la figure 2 représente une vue de ce dispositif libéré ;
- la figure 3 représente une vue d'un dispositif selon une variante de la figure 2 ;
- la figure 4 représente une vue en section d'un autre dispositif selon l'invention en cours de localisation ;
- la figure 5 représente une vue en section d'une étape intermédiaire de l'implantation ;
- la figure 6 représente une vue en section de ce dispositif à l'état implanté ;
- la figure 7 représente une vue en section de l'extrémité d'un autre dispositif selon l'invention en cours de localisation ;
- la figure 8 représente une vue de ce dispositif au moment de l'implantation ;
- la figure 9 représente une vue du dispositif une fois implanté ;
- la figure 10 représente une vue de deux dispositifs implantés dans une arborisation vasculaire ; et
- les figures 11a, 11b, 11c représentent une vue schématique en coupe transversale de différentes formes possible de la section de la tige allongée du dispositif.

Comme représenté schématiquement sur le dessin, un vaisseau (1) présente une première zone (2) dans laquelle la paroi vasculaire est normale compte tenu de l'état et de l'âge du patient, suivie d'un tronçon (3) dans lequel la paroi a subi une angioplastie ramenant la lumière (4) de ce vaisseau à un diamètre suffisant.

Le dispositif (5) selon l'invention est contenu à l'extrémité d'un cathéter intravasculaire (6) à l'intérieur duquel peut coulisser un fil ou jonc de guidage et d'intervention classique (7) placé dans l'alignement du dispositif (5) L'autre extrémité, non représentée, du cathéter et du fil (7) est située à l'extérieur du corps du patient et munie de moyens de manoeuvre habituels, tels que décrits par exemple dans les demandes de brevet précitées. On comprend donc que si on laisse le guide (7) dans sa position et que l'on retire le cathéter (6) vers la gauche, le cathéter va libérer progressivement le dispositif (5).

Le dispositif (5) est réalisé, par exemple, en métal et présente un élément allongé (8) dont une extrémité est libre et, raccordés à l'élément allongé (8), des moyens (9) comportant une première partie rectiligne (10) prolongeant l'élément (8) et aboutissant, après un coude (11), à un bras antérieur libre (12). Au niveau du coude s'étend une autre partie (13) sous forme d'un embranchement se terminant par un bras libre (14) à la suite d'un coude (15).

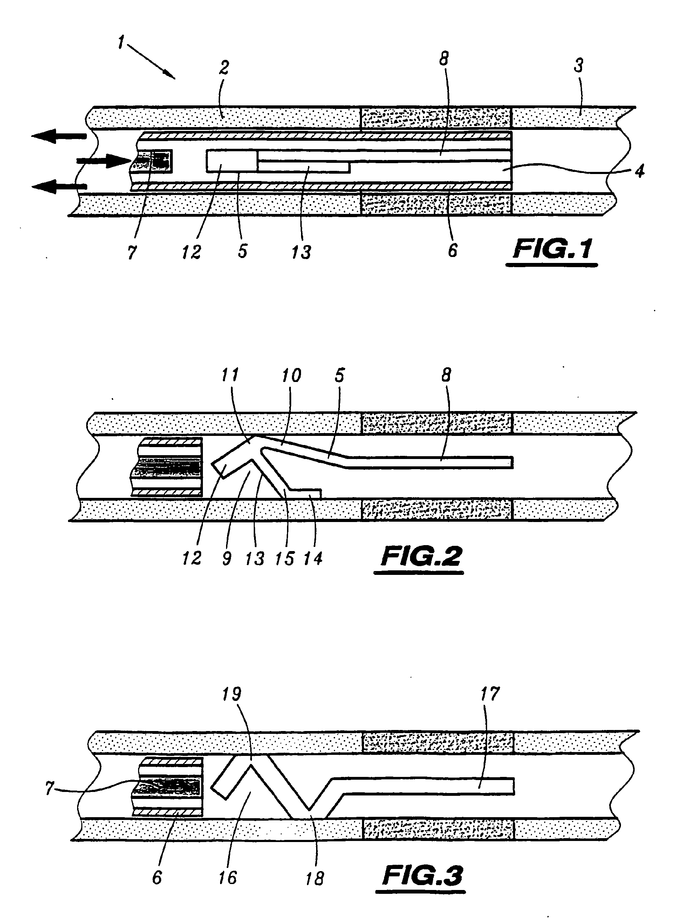

Ce dispositif est semi-rigide et occupe, lorsqu'il est emprisonné à l'intérieur du cathéter (6), une forme sensiblement rectiligne dans laquelle les prolongements (10, 13) et les pattes (12, 14) sont peu ou pas inclinés par rapport à l'élément allongé cylindrique (8). Lorsqu'on libère le dispositif, il prend spontanément la forme représentée sur la figure 2 dans laquelle la partie (5) est oblique par rapport au prolongement (8) jusqu'au coude (11) tandis que la branche (13) s'écarte à l'opposé du coude (11) pour venir au contact de la paroi vasculaire par le coude (15) et la patte (14) qui le prolonge. La patte (12) antérieure s'incline à nouveau vers l'avant et vers le centre de la lumière vasculaire et forme ainsi un élément susceptible d'être repris par un dispositif convenable d'extraction.

On comprend que dans cette forme de réalisation, lorsque le dispositif est libéré, les coudes (11) et (15) vont venir au contact, en des points situés dans un plan diamétral opposé, de la paroi vasculaire, et grâce à leur élasticité résiduelle, vont maintenir le dispositif dans sa position dans laquelle l'élément allongé (8) est séquent dans la lumière au niveau du tronçon (3).

Par un choix adéquat et conforme à l'invention, du volume occupé par le prolongement (8), on provoque dans la zone qui l'entoure une variation de vitesse du liquide circulant dans la lumière vasculaire et, par conséquent, l'effet thérapeutique recherché.

En se référant à la figure 3, on voit une variante plus simple du dispositif selon l'invention, dans lequel le dispositif (16) présente l'élément allongé en forme de tige libre (17) suivi d'un moyen d'ancrage ayant une forme de S déterminant deux coudes (18, 19) par lesquels le dispositif va s'appuyer et se maintenir contre la paroi vasculaire. Ce dispositif a une forme pratiquement rectiligne lorsqu'il est contre l'intérieur du cathéter et se déploie pour prendre la forme représentée sur le dessin lorsqu'il a été libéré par le même procédé que celui décrit à l'occasion des figures 1 et 2.

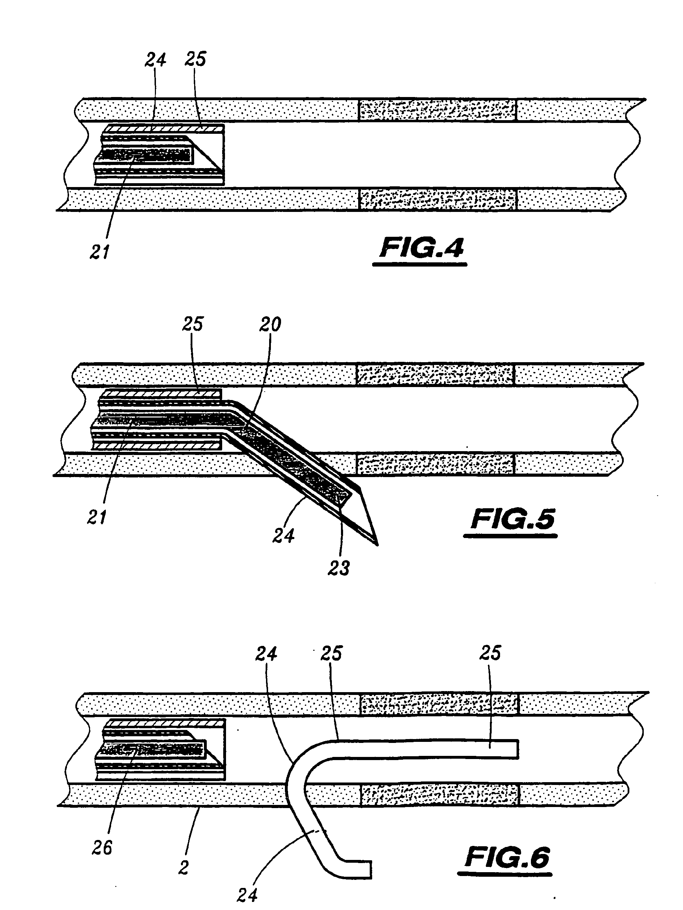

En se référant maintenant aux figures 4 à 6, on voit un dispositif selon une autre forme de réalisation. Ce dispositif (20) présente une tige allongée (21) qui se poursuit par un moyen d'ancrage (22) obtenu par une déformation en forme de coude dont une partie, (23), traverse la paroi vasculaire (2).

Pour la mise en place d'un tel dispositif, on insère le dispositif semi-rigide à l'intérieur d'une aiguille d'implantation (24) contenue dans un cathéter (25). Cette aiguille est d'un type connu qui, lorsqu'elle sort suffisamment du cathéter (24) en étant repoussée par les moyens de commande situés à l'extérieur du corps, forme un coude qui la dirige obliquement par rapport à la lumière vasculaire jusqu'à venir traverser la paroi vasculaire comme on le voit sur la figure 5. Une fois que l'aiguille est dans cette position, on maintient, par un fil-guide non représenté, le dispositif (20) dans cette position puis on rétracte l'aiguille (24) à l'intérieur du cathéter (25), de sorte que le dispositif reste en place en étant ancré dans la paroi vasculaire par traversée de la paroi comme représenté sur la figure 6, l'élasticité propre du dispositif lui faisant prendre la forme représentée sur la figure 6.

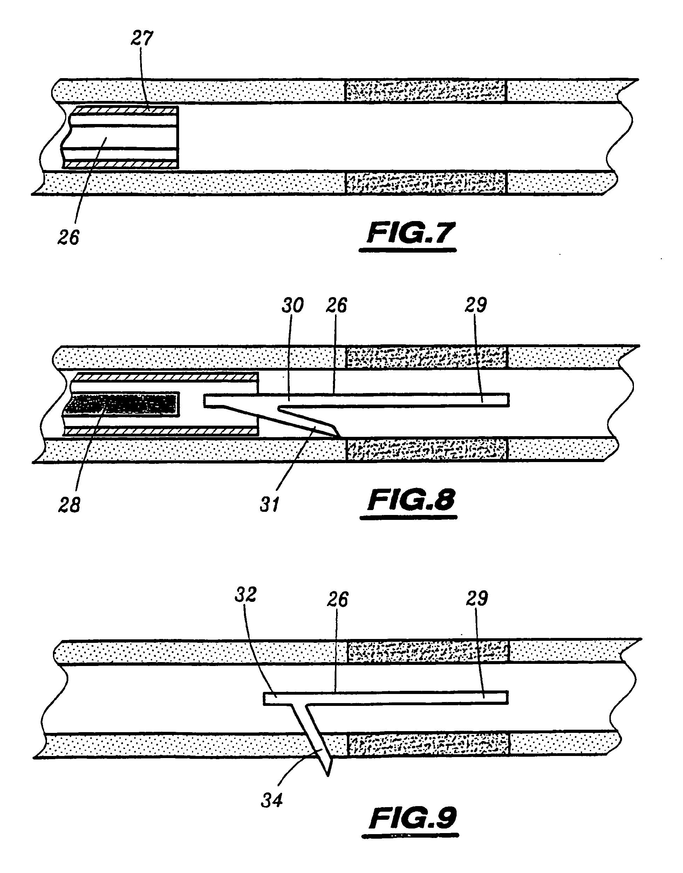

En se référant maintenant aux figures 7 à 9, on voit un dispositif selon une autre forme de réalisation de l'invention. Ce dispositif (26) est initialement contenu et contraint dans un cathéter (27) d'où il peut être extrait, le cathéter restant fixe, par un guide (28) coulissant dans le cathéter. Le dispositif comporte également une tige allongée (29) et des moyens d'ancrage (30) comprenant un bras (31) se terminant en pointe à la façon d'un ardillon. Lorsque le dispositif est libéré, ce bras s'écarte obliquement du reste, rectiligne, du dispositif et, sous la poussée du fil-guide (28), son extrémité pointue va pénétrer dans la paroi vasculaire où elle s'accroche. L'extrémité antérieure (32) du dispositif est également rectiligne et prolonge la tige (29) pour former une zone de liaison lui permettant d'être pris par un moyen de préhension, tel que par exemple une pince, amené par un cathéter, lorsque l'on veut extraire le dispositif.

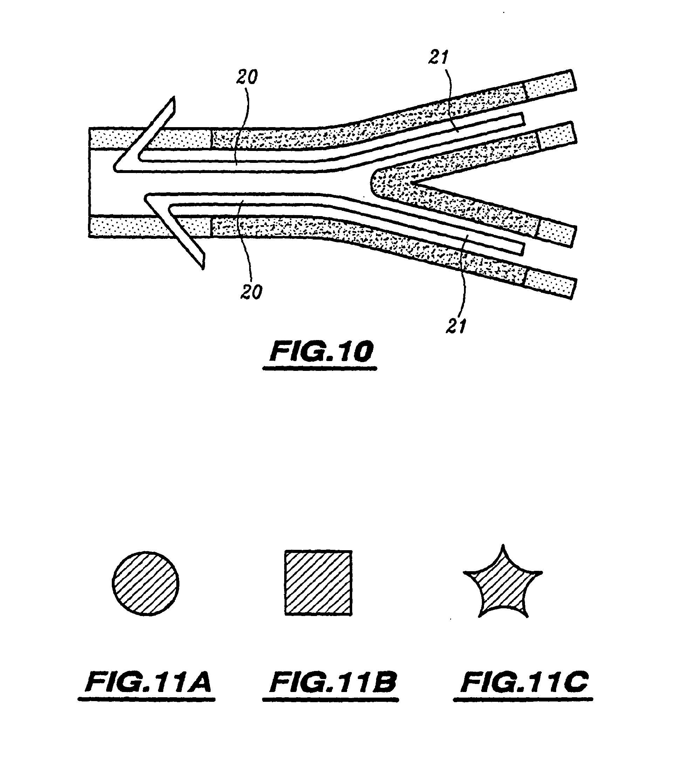

En se référant à la figure 10, on voit deux dispositifs (20) selon la forme de réalisation schématisée par la figure 6 installés de façon à ce que chacune des deux tiges allongées (21) se trouve dans une des voies vasculaires à traiter.

En se référant maintenant à la figure 11, on voit trois formes possibles du dispositif correspondant à la section dudit dispositif par exemple dans la partie de la tige allongée.

L'invention ne se limite pas aux dispositifs précités mais entend couvrir tous les dispositifs de forme pleine allongée, de surface lisse et de section et diamètre constants ou non directement maintenus ou accrochés à la paroi du vaisseau en dehors de la zone de sténose ou, par l'intermédiaire d'une zone d'ancrage par contact ou insertion. La zone d'ancrage par contact sera supportée par les parois du vaisseau en au moins deux points d'appui. La zone d'ancrage par insertion n'aura qu'un seul point de contact. Dans les deux cas, aucun effet stent, d'écartement des parois vasculaires, n'est recherché.

Le dispositif sera utilisable dans les vaisseaux sanguins et dans tous les conduits contenant un fluide biologique où l'utilisation du stent est possible et où il existe un endothélium susceptible de réagir à la circulation locale.

## Revendications

1. Dispositif implantable dans les voies vasculaires, comportant un élément intraluminal (8, 17, 21, 29) allongé susceptible d'occuper, dans un tronçon de la lumière vasculaire, un volume de cette lumière, ledit élément étant associé à un moyen de fixation dans la lumière vasculaire,
**caractérisé par** une extrémité proximale ou zone d'ancrage (12, 13, 18, 19, 23, 31) susceptible de se fixer sur ou à travers, la paroi du vaisseau, suivie d'une tige ayant une extrémité distale libre dans le courant sanguin de telle sorte que seule l'extrémité proximale ou la zone d'ancrage soit en contact permanent avec les tissus du vaisseau,
cette tige et cette extrémité distale formant l'élément intraluminal en ne diminuant pas le flux sanguin et n'exerçant pas d'action mécanique sensible sur la paroi vasculaire.

2. Dispositif selon la revendication 1, **caractérisé en ce que** l'élément intraluminal est une tige pleine (8, 17, 21, 29) de forme allongée, de surface lisse, de diamètre et de sections constants.

3. Dispositif selon la revendication 1 ou 2 de 0,5 à 2 mm de diamètre et de 0,5 à 15 cm de longueur.

4. Dispositif selon la revendication 1 ou 2 de section comprise entre 6 et 50 % de la section du vaisseau où il est implanté.

5. Dispositif selon la revendication 1 ou 2 de section comprise entre 10 et 20 % de la section du vaisseau où il est implanté.

6. Dispositif selon l'une des revendications 1 à 5 de section cylindrique, ovale, carrée, en étoile ou en fleur.

7. Dispositif selon l'une des revendications 1 à 6 dans lequel ledit moyen de fixation comprend un zone d'ancrage réalisée en un matériau permettant, à la pose du dispositif dans la lumière vasculaire, un changement de forme entraînant au moins deux points de contact avec la paroi vasculaire,

8. Dispositif selon l'une des revendications 1 à 6 dans lequel ledit moyen de fixation (23, 31) comprend une zone d'ancrage permettant directement son implantation dans la paroi du vaisseau.

9. Dispositif salon la revendication 8, **caractérisé en ce que** ladite zone d'ancrage est réalisée en un matériau permettant sa déformation, à la pose du dispositif dans la lumière vasculaire.

10. Dispositif selon l'une des revendications 7 à 9, **caractérisé en ce qu'**il est déformable dans une position sensiblement rectiligne pour sa mise en place par l'intermédiaire d'une aiguille ou d'un cathéter.

11. Dispositif selon les revendications précédentes dans lequel lesdits moyens de fixation sont agencés pour permettre le retrait du dit dispositif.

12. Dispositif selon l'une des revendications précédentes réalisé en acier inox, en alliage, ou en plastique.

13. Dispositif selon l'une des revendications 1 à 11 réalisé en Nylon, polysulfone, en Téflon ou en Silicone.

14. Dispositif selon l'une des revendications 1 à 11 réalisé en PLGA.

15. Dispositif selon l'une des revendications précédentes dont la surface est recouverte d'un revêtement.

16. Dispositif selon la revendication 15 dans lequel le dit revêtement est en acide hyaluronique, en collagène, en PEG, en glycol, en hydrogel ou en PLGA.

17. Dispositif selon l'une des revendications précédentes contenant un principe actif libérable.

18. Dispositif selon la revendication 17 dans lequel le principe actif est le Lanréotide, l'hydrocortisone, un glucocorticoïde ou un cytostatique.

19. Dispositif selon la revendication 12 dont la surface est hydrophile, siliconisée ou épilamée.

20. Dispositif selon les revendications précédentes réalisé par extrusion ou co-extrusion fondue ou par filage et étirage.

## Claims

1. Device that can be implanted in the vasculature, comprising an elongate intraluminal element (8, 17, 21, 29) capable, in a portion of the vascular lumen, of occupying a volume of this lumen, said element being associated with a means of fixing into the vascular lumen,
**characterized by** a proximal end or anchoring zone (12, 13, 18, 19, 23, 31) that can be fixed to or through the wall of the vessel, followed by a rod having a distal end which is free in the blood stream so that only the proximal end or the anchoring zone is in permanent contact with the tissues of the vessel,
this rod and this distal end forming the intraluminal element not reducing the blood flow and not exerting any appreciable mechanical action on the vascular wall.

2. Device according to Claim 1, **characterized in that** the intraluminal element is a solid rod (8, 17, 21, 29) of elongate shape, with a smooth surface, and with constant diameter and cross section.

3. Device according to Claim 1 or 2, with a diameter of 0.5 to 2 mm and a length of 0.5 to 15 cm.

4. Device according to Claim 1 or 2, with a cross section of between 5 and 50% of the cross section of the vessel in which it is implanted.

5. Device according to Claim 1 or 2, with a cross section of between 10 and 20% of the cross section of the vessel in which it is implanted.

6. Device according to one of Claims 1 to 5, of cylindrical, oval, square, star-shaped or flower-shaped cross section.

7. Device according to one of Claims 1 to 6, in which said fixing means comprises an anchoring zone made of a material which, when the device is placed in the vascular lumen, allows a change in shape leading to at least two points of contact with the vascular wall.

8. Device according to one of Claims 1 to 6, in which said fixing means (23, 31) comprises an anchoring zone allowing direct implantation in the wall of the vessel.

9. Device according to Claim 8, **characterized in that** said anchoring zone is made of a material allowing it to deform when the device is placed in the vascular lumen.

10. Device according to one of Claims 7 to 9,
**characterized in that** it is deformable into a roughly straight position so that it can be fitted using a needle or a catheter.

11. Device according to the preceding claims, in which said fixing means are arranged to allow said device to be withdrawn.

12. Device according to one of the preceding claims, made of stainless steel, alloy or plastic.

13. Device according to in one of Claims 1 to 11, made of nylon, polysulfone, of Teflon or of silicone.

14. Device according to in one of Claims 1 to 11, made of PLGA.

15. Device according to one of the preceding claims, the surface of which is covered with a coating.

16. Device according to Claim 15, in which said coating is made of hyaluronic acid, collagen, PEG, glycol, hydrogel or PLGA.

17. Device according to one of the preceding claims, containing a releasable active principle.

18. Device according to Claim 17 in which the active principle is Lanreotide, hydrocortisone, a glucocorticoid or a cytostatic.

19. Device according to Claim 12, the surface of which is hydrophilic, silicone coated or epilam-coated.

20. Device according to the preceding claims produced by melt extrusion or co-extrusion or by die-forming and drawing.

## Patentansprüche

1. Vorrichtung zum Einsetzen in Gefäßgänge, umfassend ein längliches intraluminales Element (8, 17, 21, 29), das in einem Abschnitt der Gefäßöffnung ein Volumen dieser Öffnung einnehmen kann, wobei das Element mit einem Mittel zur Befestigung in der Gefäßöffnung verbunden ist,
**gekennzeichnet durch** ein proximales Ende oder eine Verankerungszone (12, 13, 18, 19, 23, 31), die auf der Wand des Gefäßes oder **durch** die Wand des Gefäßes befestigt werden kann, gefolgt von einer Stange mit einem freien distalen Ende im Blutstrom, so dass nur das proximale Ende oder die Verankerungszone mit den Geweben des Gefäßes in ständigem Kontakt ist,
wobei diese Stange und dieses distale Ende das intraluminale Element bilden, wobei sie den Blutstrom nicht verringern und keine spürbare mechanische Wirkung auf die Gefäßwand ausüben.

2. Vorrichtung nach Anspruch 1, **dadurch gekennzeichnet, dass** das intraluminale Element eine volle Stange (8, 17, 21, 29) von länglicher Form, glatter Oberfläche und konstantem Durchmesser und Querschnitt ist.

3. Vorrichtung nach Anspruch 1 oder 2 von 0,5 bis 2 mm Durchmesser und 0,5 und 15 cm Länge.

4. Vorrichtung nach Anspruch 1 oder 2 mit einem Querschnitt zwischen 5 und 50 % des Querschnittes des Gefäßes, in das sie eingesetzt ist.

5. Vorrichtung nach Anspruch 1 oder 2, mit einem Querschnitt zwischen 10 und 20 % des Querschnittes des Gefäßes, in das sie eingesetzt ist.

6. Vorrichtung nach einem der Ansprüche 1 bis 5 mit zylindrischem, ovalem, quadratischem, sternförmigem oder blütenartigem Querschnitt.

7. Vorrichtung nach einem der Ansprüche 1 bis 6, bei der das Befestigungsmittel eine Verankerungszone umfasst, die aus einem Material ausgeführt ist, das bei der Anbringung der Vorrichtung in der Gefäßöffnung eine Formänderung ermöglicht, die zu mindestens zwei Kontaktpunkten mit der Gefäßwand führt.

8. Vorrichtung nach einem der Ansprüche 1 bis 6, bei der das Befestigungsmittel (23, 31) eine Verankerungszone umfasst, die direkt ihr Einsetzen in die Gefäßwand ermöglicht.

9. Vorrichtung nach Anspruch 8, **dadurch gekennzeichnet, dass** die Verankex-ungszone aus einem Material hergestellt ist, das ihre Verformung bei der Anbringung der Vorrichtung in der Gefäßöffnung ermöglicht.

10. Vorrichtung nach einem der Ansprüche 7 bis 9, **dadurch gekennzeichnet, dass** sie in eine im Wesentlichen gerade Position für ihre Anbringung mit Hilfe einer Nadel oder eines Katheters verformbar ist.

11. Vorrichtung nach den vorhergehenden Ansprüchen, bei der die Befestigungsmittel derart angeordnet sind, dass sie das Herausziehen der Vorrichtung ermöglichen.

12. Vorrichtung nach einem der vorhergehenden Ansprüche, die aus rostfreiem Stahl, einer Legierung oder Kunststoff hergestellt ist.

13. Vorrichtung nach einem der Ansprüche 1 bis 11, die aus Nylon, Polysulfon, Teflon oder Silikon hergestellt ist.

14. Vorrichtung nach einem der Ansprüche 1 bis 11, die aus PLGA hergestellt ist.

15. Vorrichtung nach einem der vorhergehenden Ansprüche, deren Oberfläche mit einer Beschichtung überzogen ist.

16. Vorrichtung nach Anspruch 15, bei der die Beschichtung aus Hyaluronsäure, Kollagen, PEG, Glykol, Hydrogel oder PLGA ist.

17. Vorrichtung nach einem der vorhergehenden Ansprüche, die ein freisetzbares Aktivprinzip enthält.

18. Vorrichtung nach Anspruch 17, bei der das Aktivprinzip das Lanreotid, Hydrokortison, ein Glukokortikoid oder ein zytostatikum ist.

19. Vorrichtung nach Anspruch 12, deren Überfläche hydrophil, mit Silikon oder Epilamen überzogen ist.

20. Vorrichtung nach den vorhergehenden Ansprüchen, die durch Extrusion oder Co-Extrusion oder durch Ziehen und Strecken hergestellt ist.
